COMPLETE DOCUMENT

(19) Europäisches Patentamt
European Patent Office
Office européen des brevets

(11) Publication number: 0 048 168
A2

(12) EUROPEAN PATENT APPLICATION

(21) Application number: 81304241.3

(22) Date of filing: 16.09.81

(51) Int. Cl.[3]: C 07 D 501/36
A 61 K 31/545
//C07D257/04

(30) Priority: 17.09.80 US 187860

(43) Date of publication of application:
24.03.82 Bulletin 82/12

(84) Designated Contracting States:
AT BE CH DE FR GB IT LI LU NL SE

(71) Applicant: ELI LILLY AND COMPANY
307, East McCarty Street
Indianapolis Indiana 46285(US)

(72) Inventor: Kainer, Allen Samuel
219, West 81st Street
Indianapolis Indiana 46260(US)

(74) Representative: Hudson, Christopher Mark et al,
Erl Wood Manor
Windlesham Surrey GU20, 6PH(GB)

(54) **7-Acylamino cephalosporin compounds, their preparation and pharmaceutical formulations containing them.**

(57) 7-Acylamino cephalosporins compounds are described. They have the following formula

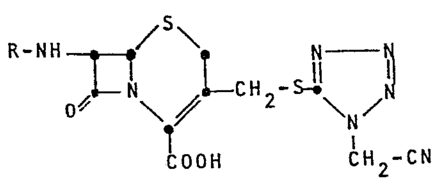

1

wherein R is hydrogen or an acyl group of the formula

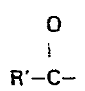

wherein R' is hydrogen, $C_1$-$C_4$ alkyl, or $C_1$-$C_4$ alkyl substituted by halogen or cyano;
or R' is a group of the formula

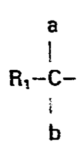

wherein $R_1$ is phenoxy, phenylthio, phenyl, hydroxyphenyl, halophenyl, halo-substituted-hydroxyphenyl, or $C_1$-$C_4$ alkylphenyl, thienyl, furyl, or 2-aminothiazol-4-yl,
a is hydrogen;
b is hydrogen, hydroxy or amino, and a and b when taken together form an oximino group of the formula

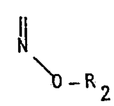

wherein $R_2$ is hydrogen, $C_1$-$C_4$ alkyl or a group of the formula

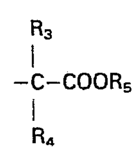

wherein $R_3$ and $R_4$ independently are hydrogen, $C_1$-$C_4$ alkyl, or $R_3$ and $R_4$ when taken together with the carbon atom to which they are attached form a four, five or six-membered carbocyclic ring;

./...

EP 0 048 168 A2

Croydon Printing Company Ltd.

$R_5$ is hydrogen or a carboxy protecting group; and the pharmaceutically acceptable salts thereof.

The compounds are broad spectrum antibiotics and are prepared by reacting the appropriate 3-substituted acyloxy or halo intermediate with 1-cyanomethyl-1H-tetrazole-5-thiol.

# 7-ACYLAMINO CEPHALOSPORIN COMPOUNDS, THEIR PREPARATION AND PHARMACEUTICAL FORMULATIONS CONTAINING THEM

This invention relates to cephalosporin antibiotic compounds. In particular it relates to broad spectrum cephalosporin antibiotics substituted with a 1-cyanomethyl-1H-tetrazol-5-ylthiomethyl group represented by the following general formula,

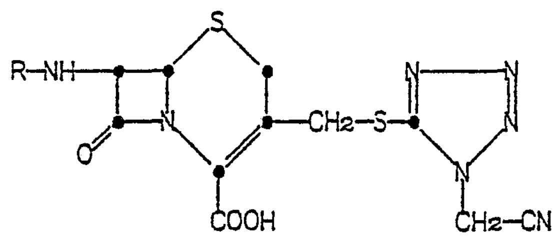

I

wherein R is hydrogen or an acyl group derived from a carboxylic acid, and to the pharmaceutically acceptable salts thereof.

The cyanomethyltetrazole substituted cephalosporins can be prepared by reacting a 7-acylamino-3-acetoxymethyl cephalosporin or 7-aminocephalosporanic acid with 1-cyanomethyl-1H-tetrazole-5-thiol. Alternatively, they can be prepared by the reaction of a 7-acylamino-3-halomethyl substituted cephalosporin with the 1-cyanomethyl-1H-tetrazole-5-thiol.

These cephalosporin antibiotics exhibit high antibacterial activity against both gram-positive and gram-negative bacteria.

A number of cephalosporin antibiotics which bear a heterocyclicthiomethyl group in the 3-position of the 3-cephem bicyclic ring system are known. Among such heterocyclic groups is the 5-membered tetrazolthiomethyl group and variously substituted tetrazolthiomethyl substituent groups. For example, Takano et al., U.S. Patent 3,516,997 teach 1H-tetrazolthiomethyl and 1-methyl-1H-tetrazolthiomethyl substituted compounds, notably the antibiotic known as cefazolin. Ryan, U.S. Patent 3,641,021 describes cephalosporin antibiotics wherein the 3-position is substituted with a 1-($C_1$-$C_3$)-1H-tetrazolthiomethylgroup. In particular, Ryan describes the clinically useful antibiotic known as cephamandole, 7-D-mandelamido-3-(1-methyl-1H-tetrazolthiomethyl)-3-cephem-4-carboxylic acid. Berges, U.S. Patent 4,101,656 describes cephalosporins having a tetrazolthiomethyl substituent which is substituted with an alkylsulfonamidoalkyl group. U.S. Patent 4,100,346 teaches cephalosporin antibiotics substituted with 1-carboxyethyl and 1-carboxypropyl-1H-tetrazolthiomethyl substituents. British Patent Specification No. 1,525,626 teaches certain cephalosporin compounds having a 1-carboxymethyl-1H-tetrazolthiomethyl substituent in the 3-position.

Intensive research continues with the cephalosporin antibiotics because of the desire to synthesize antibiotics even more potent than those in clinical use. The cephalosporin compounds provided by this invention exhibit antibacterial activity against a broad spectrum of microorganisms infectious to man and animals. They differ structurally from the known cephalosporin compounds by possessing as part of their structure a cyanomethyl substituted-1H-tetrazolthiomethyl substituent in the 3-position of the cephem ring structure.

The cephalosporin compounds of this invention are represented by the following structural formula I

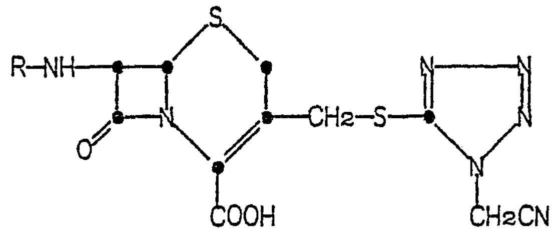

I

wherein R is hydrogen or an acyl group of the formula

$$R'-\overset{\overset{O}{\|}}{C}-$$

wherein R' is hydrogen, $C_1$-$C_4$ alkyl, or $C_1$-$C_4$ alkyl substituted by cyano or halogen; a group of the formula

$$R_1-\underset{b}{\overset{a}{C}}-$$

wherein $R_1$ is phenoxy, phenylthio, phenyl, hydroxyphenyl, halophenyl, halo-substituted-hydroxyphenyl, $C_1$-$C_4$ alkylphenyl, thienyl, furyl, or 2-aminothiazol-4-yl;

a is hydrogen;

b is hydrogen, hydroxy, or amino;

and a and b when taken together form an oximino group of the formula

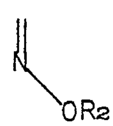

wherein $R_2$ is hydrogen, $C_1$-$C_4$ alkyl or a group of the formula

$$-\overset{R_3}{\underset{R_4}{\overset{|}{\underset{|}{C}}}}-COOR_5$$

wherein $R_3$ and $R_4$ independently are hydrogen, $C_1$-$C_4$ alkyl, or $R_3$ and $R_4$ when taken together with the carbon atom to which they are attached form a four, five or six-membered carbocyclic ring; $R_5$ is hydrogen or a carboxy protecting group;

and the pharmaceutically acceptable, non-toxic salts thereof.

As used in the above formula I the following terms have the indicated meanings: "$C_1$-$C_4$ alkyl", means methyl, ethyl, n-propyl, iso-propyl, n-butyl, t-butyl, and like straight or branched chain lower alkyl groups; "halogen" refers to fluoro, chloro or bromo; "$C_1$-$C_4$ alkyl substituted by cyano or halogen" refers to cyanomethyl, 2-cyanoethyl, 1-cyanoethyl, 3-cyanopropyl, 4-cyanobutyl, chloromethyl, bromomethyl, 2-bromoethyl, 1-chloroethyl, 2-chloropropyl, 4-bromobutyl, and the like; "hydroxyphenyl" refers to the mono- or di-hydroxyphenyl groups such as 4-hydroxyphenyl, 3-hydroxyphenyl, 2-hydroxyphenyl, 2,4-dihydroxyphenyl, 3,4-dihydroxyphenyl, and the like; "halophenyl" refers to the mono- or di-halophenyl groups such as 4-chlorophenyl, 3,4-dichlorophenyl, 3-bromophenyl, 4-fluorophenyl, 3-fluorophenyl, and the like; "halo-substituted-hydroxyphenyl" refers to halo-substituted-mono- or dihydroxyphenyl groups such as 3-chloro-4-hydroxyphenyl, 4-chloro-3,5-dihydroxyphenyl, 4-hydroxy-3,5-dichlorophenyl, 3-fluoro-4-hydroxyphenyl, 2-fluoro-4-hydroxyphenyl, 3-bromo-4-hydroxyphenyl, and the like; "$C_1$-$C_4$ alkylphenyl" refers to the mono- or di-lower alkylphenyl groups such as 2-, 3-, or 4-methylphenyl, 4-ethylphenyl, 3,4-dimethylphenyl, 4-t-butylphenyl, 4-n-propylphenyl, and the like, such alkylphenyl groups can be optionally substituted with hydroxy, or halogen groups; and "thienyl" and "furyl" refer to the respective 2- and 3-isomers thereof.

The following compounds are examples of compounds of the invention represented by the formula I:

7-formamido-3-(1-cyanomethyl-1H-tetrazol-5-ylthiomethyl)-3-cephem-4-carboxylic acid,

7-acetamido-3-(1-cyanomethyl-1H-tetrazol-5-ylthiomethyl)-3-cephem-4-carboxylic acid,

7-cyanoacetamido-3-(1-cyanomethyl-1H-tetrazol-5-ylthiomethyl)-3-cephem-4-carboxylic acid,

7-bromoacetamido-3-(1-cyanomethyl-1H-tetrazol-5-ylthiomethyl)-3-cephem-4-carboxylic acid,

7-phenylacetamido-3-(1-cyanomethyl-1H-tetrazol-5-ylthiomethyl)-3-cephem-4-carboxylic acid,

7-phenoxyacetamido-3-(1-cyanomethyl-1H-tetrazol-5-ylthiomethyl)-3-cephem-4-carboxylic acid,

7-phenylmercaptoacetamido-3-(1-cyanomethyl-1H-tetrazol-5-ylthiomethyl)-3-cephem-4-carboxylic acid,

7-(2-thienylacetamido)-3-(1-cyanomethyl-1H-tetrazol-5-ylthiomethyl)-3-cephem-4-carboxylic acid,

7-(2-furylacetamido)-3-(1-cyanomethyl-1H-tetrazol-5-ylthiomethyl)-3-cephem-4-carboxylic acid; and when in formula 1, b is hydroxy or amino,

7-mandelamido-3-(1-cyanomethyl-1H-tetrazol-5-ylthiomethyl)-3-cephem-4-carboxylic acid,

7-[α-hydroxy-α-(2-thienyl)acetamido]-3-(1-cyanomethyl-1H-tetrazol-5-ylthiomethyl)-3-cephem-4-carboxylic acid,

7-phenylglycylamino-3-(1-cyanomethyl-1H-tetrazol-5-ylthiomethyl)-3-cephem-4-carboxylic acid, and when in formula 1, a and b are taken together to form an oximino group,

7-[α-methoximino-α-(2-amino-1,3-thiazol-4-yl)-acetamido]-3-(1-cyanomethyl-1H-tetrazol-5-ylthiomethyl)-3-cephem-4-carboxylic acid,

7-(α-ethoxyimino-phenylacetamido)-3-(1-cyanomethyl-1H-tetrazol-5-ylthiomethyl-3-cephem-4-carboxylic acid,

7-[α-methoxyimino-α-(2-furyl)acetamido]-3-(1-cyanomethyl-1H-tetrazol-5-ylthiomethyl)-3-cephem-4-carboxylic acid,

7-[α-isopropoxyimino-α-(2-amino-1,3-thiazole-4-yl)acetamido]-3-(1-cyanomethyl-1H-tetrazole-5-ylthiomethyl)-3-cephem-4-carboxylic acid,

7-[α-methoxyimino-α-(2-thienyl)acetamido]-3-(1-cyanomethyl-1H-tetrazole-5-ylthiomethyl)-3-cephem-4-carboxylic acid,

7-[α-(2-carboxyprop-2-yloxyimino)-α-(2-amino-1,3-thiazol-4-yl)acetamido]-3-(1-cyanomethyl-1H-tetrazol-5-ylthiomethyl)-3-cephem-4-carboxylic acid, and

7-[α-(carboxycyclobut-1-yloxyimino)-α-(2-amino-1,3-thiazol-4-yl)acetamido]-3-(1-cyanomethyl-1H-tetrazol-5-ylthiomethyl)-3-cephem-4-carboxylic acid

The cephalosporin compounds of this invention have the conventional stereochemical configuration of the known cephalosporins. For example, the 7-position side chain is β; the carbon atom in the side chain to which is attached either the amino group or the hydroxy group (b = $NH_2$ or OH, formula I) has the D-configuration; and when in formula I, a and b form an oximino group, the oxime function can have either the syn or anti configuration or a mixture of both. Preferably the oxime is in the syn form.

As with the known cephalosporin antibiotics the carboxylic acid group in the 4-position of the cephem ring system is acidic and forms salts with suitable bases. Preferred salts are the pharmaceutically acceptable non-toxic salts such as the alkali metal salts for example, the sodium or potassium salts; the calcium salt, the aluminum salt, salts formed with amines and ammonia such as the ammonium salt, the di-lower-alkylamine salts, the diethanolamine salt, the dibenzylamine salt, the cyclohexylamine salt, the dicyclohexylamine salt, the procaine salt, and like amine salts. These salts are formed by conventional procedures employed in the cephalosporin art.

The cephalosporin compounds of this invention are prepared via alternative methods. According to one method a 7-acylamino-3-acetoxymethyl-3-cephem-4-carboxylic acid represented by the formula

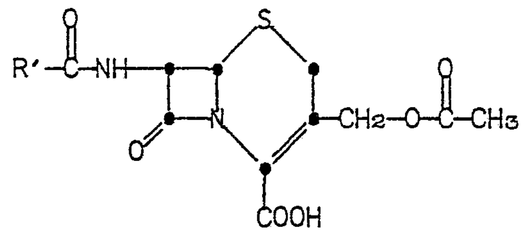

wherein R' has the same meanings as defined for formula I, is reacted with 1-cyanomethyl-1H-tetrazole-5-thiol represented by the formula

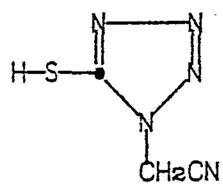

to form the compound of the formula I. The reaction can be carried out at a temperature between about 35°C. and about 75°C. under aqueous conditions at a pH of about 7 to about 9 and preferably in the presence of a water miscible organic solvent such as acetonitrile or tetrahydrofuran.

The above reaction can also be carried out under non-aqueous conditions by following the process described by Hatfield in U.S. Patent No. 4,144,391.

According to another method, 7-aminocephalosporanic acid is reacted with 1-cyanomethyl-1H-tetrazole-5-thiol and the 7-amino-3-(1-cyanomethyl-1H-tetrazole-5-ylthiomethyl)-3-cephem-4-carboxylic acid obtained is N-acylated at the 7-amino group to obtain the compound of formula I. This method is illustrated by the following reaction scheme.

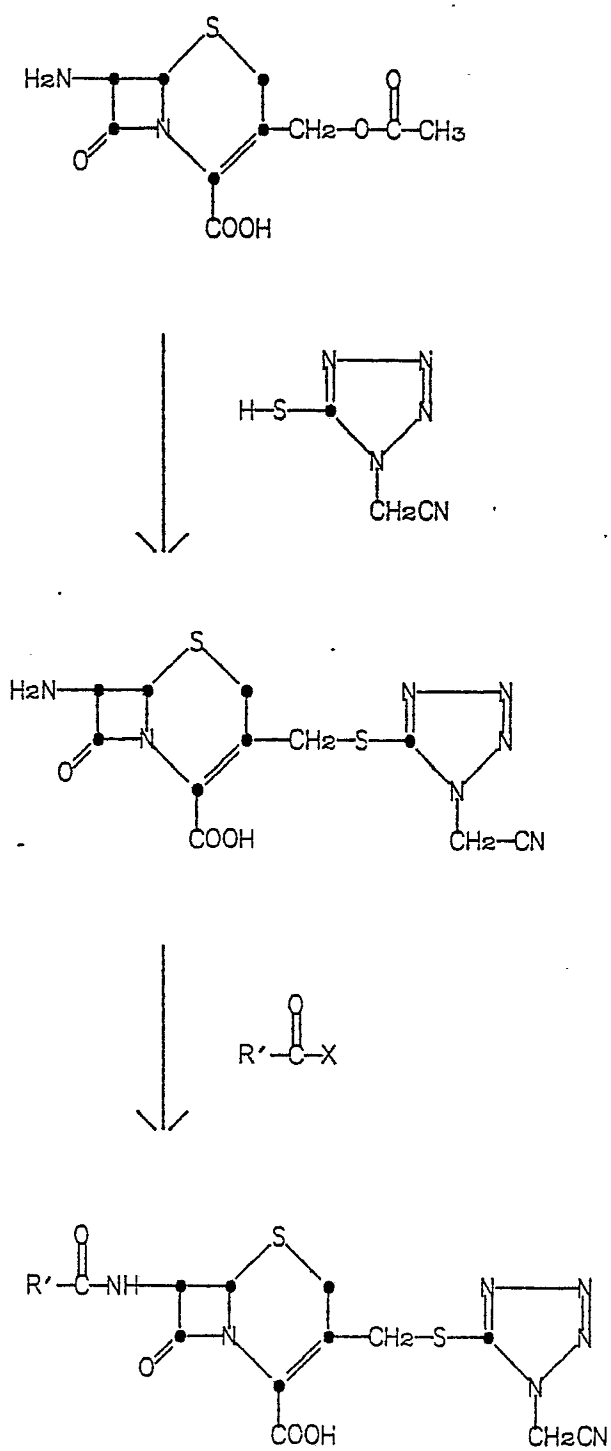

In the first step of the above sequence the acetoxy group of 7ACA is displaced by the tetrazole thiol in an aqueous medium at a pH of about 7 to 9. The displacement is best carried out at a temperature of about 60°C. although product is obtained over the range of from about 40°C. to about 75°C.

In the second step the substituted nucleus is acylated at the amino group in the 7-position with the desired carboxylic acid R'COOH to form the acylated product. In the above reaction scheme, R'-COX, refers to an active derivative of the carboxylic acid. For example, X can be chloro, bromo, azido, or an active ester forming moiety such as $-O-\overset{O}{\overset{\|}{C}}-CH_3$ or $-O-\overset{O}{\overset{\|}{C}}-$isobutyl, formed with methyl or isobutyl chloroformate and the acid, or other active esters such as those formed with hydroxybenzotriazole (HBT) or hydroxysuccinimide. The acylation is carried out by employing the acylation techniques used in the acylation of other cephalosporin nuclei such as 7ACA and 7ADCA.

Alternatively, the compounds of the invention are prepared by reacting a 7-acylamino-3-halomethyl-3-cephem-4-carboxylic acid ester with 1-cyanomethyl-1H-tetrazole-5-thiol as illustrated in the following reaction scheme.

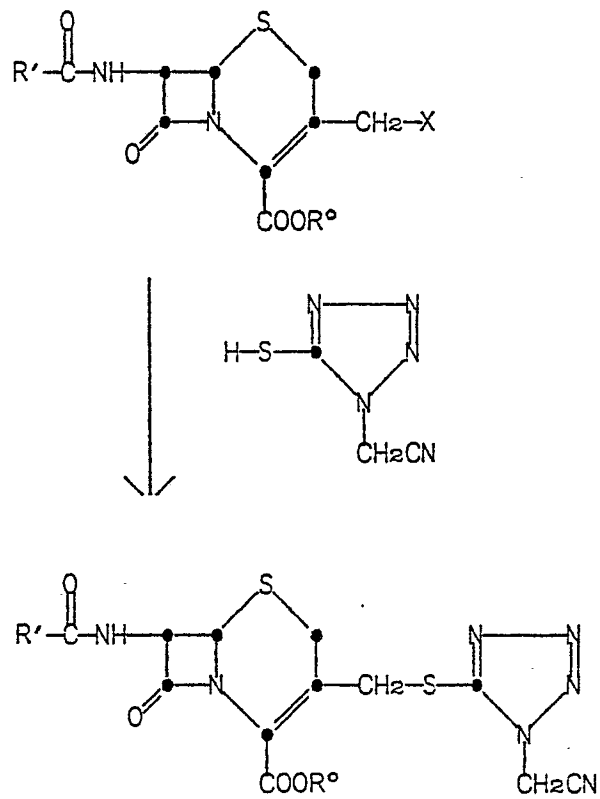

wherein X represents chloro, bromo or iodo and R° is a carboxy protecting ester group.

The reaction is preferably carried out in an inert organic solvent such as methylene chloride in the presence of a hydrogen halide acceptor such as pyridine or triethylamine. Following the reaction the ester group R° is removed via deesterification to provide the compound of formula I. In carrying out the preparation the carboxylic acid group of the starting material is protected by esterification. Ester groups which are commonly used for the temporary protection of cephalosporin acids can be used. For example, such esters include the benzyl, diphenylmethyl, p-nitrobenzyl,

p-methoxybenzyl, t-butyl, 2,2,2-trichloroethyl, 2-iodoethyl, and phenacyl esters. Silyl esters, such as trimethylsilyl can be employed with success under essentially anhydrous conditions.

The compounds of the invention are in general prepared preferably by the first of the above described methods, i.e. by the displacement of the acetoxy group of a 7-acylamino-3-acetoxymethyl-3-cephem-4-carboxylic acid with the cyanomethyltetrazole-5-thiol. The compounds of the formula I wherein R' is $C_1$-$C_4$ alkyl substituted by halogen, are prepared by acylating the 3-substituted nucleus, 7-amino-3-(1-cyanomethyl-1H-tetrazol-5-ylthiomethyl)-3-cephem-4-carboxylic acid, (formula I, R = H).

A preferred group of antibiotics of this invention are represented by the formula I wherein R is an acyl group of the formula

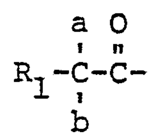

wherein a and b are taken together to form an oximino group of the formula

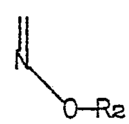 .

The preferred compounds are thus represented by the formula

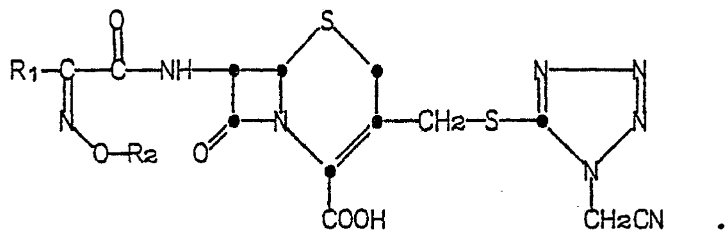

An especially preferred group of compounds are represented by the above formula when $R_1$ is the 2-amino-1,3-thiazol-4-yl group. These compounds are represented by the following structural formula

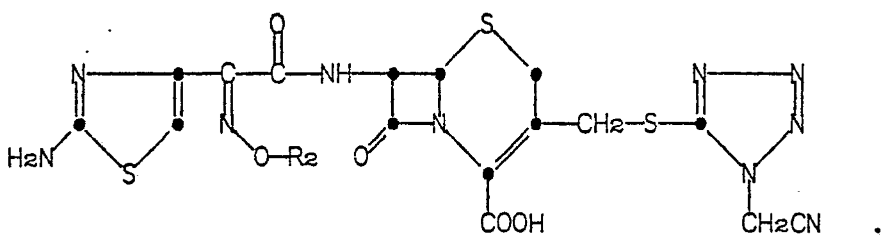

Preferably the oximino compounds of the above formula have the <u>syn</u> configuration.

The preferred compounds of the above formulas are prepared, as described previously herein, by the displacement of the acetoxy group of a 3-acetoxymethyl-3-cephem-4-carboxylic acid having the desired oximino substituted 7-acyl side chain as illustrated below.

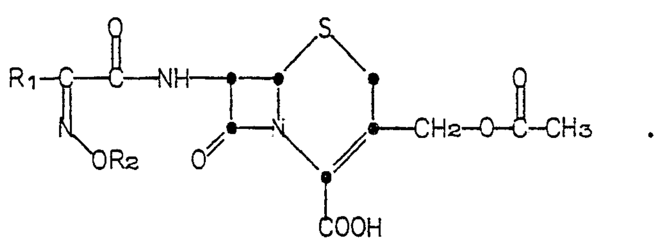

For example, when $R_1$ in the above formula is the 2-amino-1,3-thiazol-4-yl group and $R_2$ is methyl, 7β-[α-methoxyimino-α-(2-amino-1,3-thiazol-4-yl)acetamido]-3-acetoxymethyl-3-cephem-4-carboxylic acid is added to a solution of 1-cyanomethyl-1H-tetrazole-5-thiol in pH 7 buffer containing sodium bicarbonate and the solution is heated at a temperature of about 50° to about 75°C. for about 3 hours. The product is isolated and can be purified by chromatography. Reverse phase silica gel HPLC chromatography is a preferred purification technique especially useful for the separation and purification of the preferred oximino compounds of the invention wherein $R_1$ is the 2-amino-1,3-thiazol-4-yl group.

The 3-acetoxymethyl-3-cephems having the 2-amino-1,3-thiazole oxime side chain wherein $R_2$ is $C_1$-$C_4$ alkyl, which are used in the preparation of preferred compounds of this invention are described by U.S. Patent No. 4,152,432. The preferred compounds wherein $R_2$ is a carboxy substituted alkyl moiety of the formula

$$\begin{array}{l} \ R_3 \\ \ \vert \\ -C-COOR_5 \\ \ \vert \\ \ R_4 \end{array}$$

as defined above, are prepared in an analogous manner, for example by following the procedures described by U.S. Patent 4,162,360 for the preparation of the substituted oximes described therein. For example an amino-protected 2-amino-1,3-thiazol-4-ylacetic acid α-oxime of the formula

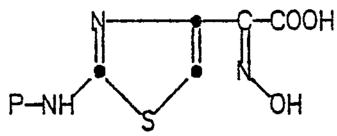

wherein P is an amino-protecting group such as an acid hydrolisable group which forms a urethane with the amino for example, t-butyloxycarbonyl, benzhydryloxy-carbonyl, and benzyloxycarbonyl, is converted with potassium t-butoxide to the salt of the oxime hydroxy group and the latter is alkylated with the appropriate carboxy-substituted alkyl halide. For example, 7-[α-(2-carboxyprop-2-yloxyimino)-α-(2-amino-1,3-thiazol-4-yl)acetamido]-3-(1-cyanomethyl-1H-tetrazol-5-ylthio-methyl)-3-cephem-4-carboxylic acid represented by the formula

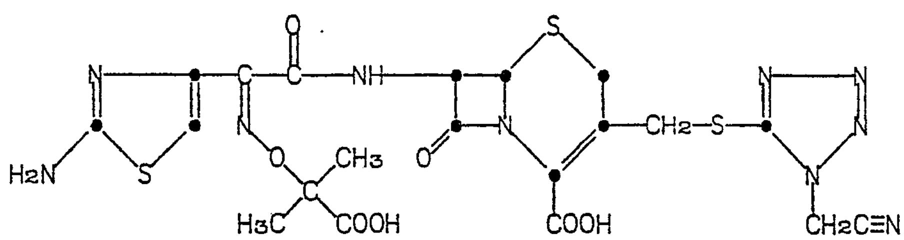

can be prepared by acylating 7-aminocephalosporanic acid with α-[2-(4-methoxybenzyloxycarbonyl)prop-2-yloximino]-2-t-butyloxycarbamido-1,3-thiazol-4-ylacetic acid to provide the esterified and amino-protected intermediate represented by the following formula

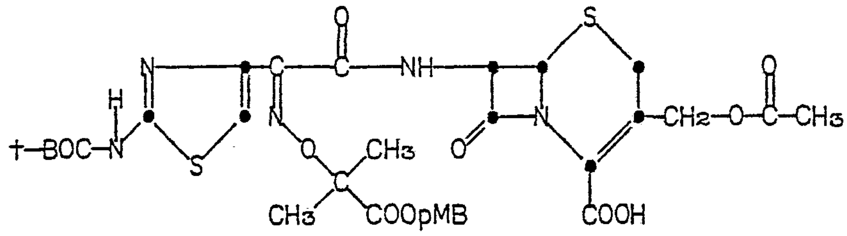

wherein t-BOC represents the t-butyloxycarbonyl group and pMB represents the p-methoxybenzyl group. The acylation is carried out by condensing the acetic acid oxime with 7-ACA in the presence of a condensing agent such as a carbodiimide, for example, dicyclohexylcarbodiimide or, alternatively the acetic acid oxime can be converted to a mixed anhydride, such as that formed with methyl or isobutyl chloroformate, and the mixed anhydride used to couple with 7-ACA.

Following the N-acylation of 7-ACA the intermediate is reacted with trifluoroacetic acid in anisole to effect the removal of the amino-protecting group and the p-methoxybenzyl ester group. After deprotection, the deprotected acylation product is reacted with 1-cyanomethyl-1H-tetrazole-5-thiol as described previously to provide the compound of the invention.

The cephalosporin compounds of this invention are broad spectrum antibiotics which are useful in combatting infections of man and animals when administered parenterally.

The antibacterial activity of the compounds of the invention is illustrated by the <u>in vitro</u> activity of a preferred compound, 7β-[α-methoxyimino-α-(2-amino-1,3-thiazol-4-yl)acetamido]-3-(1-cyanomethyl-1H-tetrazol-5-ylthiomethyl)-3-cephem-4-carboxylic acid, against a wide spectrum of microorganisms. The activity was determined by the agar dilution technique using serial dilution. The results are tabulated in Table I.

TABLE I

*In vitro* Antibacterial Activity Of 7β-[α-methoxyimino-α-(2-amino-1,3-thiazol-4-yl)-acetamido]-3-(1-cyanomethyl-1H-tetrazol-5-ylthio-methyl)-3-cephem-4-carboxylic acid

| Microorganism | Strain | Minimum Inhibitory Concentration (mcg/ml) |
|---|---|---|
| Staphylococcus aureus | X1.1 | 1 |
| Staphylococcus aureus | V41 | 4 |
| Staphylococcus aureus | X400 | 32 |
| Staphylococcus aureus | S13E | 8 |
| Staphylococcus epidermidis | EPI1 | 4 |
| Staphylococcus epidermidis | EPI2 | 8 |
| Streptococcus Group A | C203 | .015 |
| Streptococcus pneumoniae | Park | .03 |
| Streptococcus Group D | X66 | >128 |
| Streptococcus Group D | 9960 | 128 |
| Haemophilus influenzae | C.L.[1] | .03 |
| Haemophilus influenzae | 76[2] | .015 |
| Shigella sonnei | N9 | .125 |
| Escherichia coli | N10 | .25 |
| Escherichia coli | EC14 | .06 |
| Escherichia coli | TEM | .06 |
| Klebsiella sp. | KAE | 2 |
| Enterobacter aerogenes | X68 | .06 |
| Enterobacter aerogenes | C32 | .125 |
| Enterobacter aerogenes | EB17 | .25 |
| Enterobacter cloacae | EB5 | 1 |
| Enterobacter cloacae | 265A | 32 |

Table I cont.

| | | |
|---|---|---|
| Salmonella sp. | X514 | .125 |
| Salmonella sp. | 1335 | .5 |
| Pseudomonas aeruginosa | X528 | 16 |
| Pseudomonas aeruginosa | X239 | 16 |
| Pseudomonas aeruginosa | Ps18 | 32 |
| Serratia marcescens | X93 | .125 |
| Serratia marcescens | 3E3 | .25 |
| Proteus morganii | PR15 | .06 |
| Proteus inconstans | PR33 | .125 |
| Proteus rettgeri | PR7 | .06 |
| Proteus rettgeri | C24 | .125 |
| Citrobacter freundii | CF17 | 8 |
| Bordetella bronchoseptica | 16 | 64 |

1/ sensitive strain of H. influenzae

2/ resistant strain of H. influenzae

---

1-Cyanomethyl-1H-tetrazole-5-thiol used in the preparation of the compounds of the invention is obtained when ethyl azidoacetate is heated at a temperature of about 125°C. with an excess of cyanogen chloride to form ethyl 5-chloro-1H-tetrazole-1-ylacetate as a crystalline solid. The latter product is converted to the 5-thiol by heating the 5-chloro tetrazole with sodium hydrosulfide in an organic solvent such as ethyl alcohol. The reaction is carried out satisfactorily at the reflux temperature in ethyl alcohol for about 20-24 hours. The thiol is recovered by concentrating

the reaction mixture and extracting with an organic solvent, for example, ethyl acetate.

The ethyl 5-thiol-1H-tetrazole-1-ylacetate product is then converted to the amide by heating the 5-thiol ester in a mixture of concentrated ammonium hydroxide, ammonium chloride and ethyl alcohol. The amide is recovered as the ammonium salt. The amide, 5-thiol-1H-tetrazol-1-ylacetamide ammonium salt, is then dehydrated to 1-cyanomethyl-1H-tetrazol-5-thiol. The dehydration of the amide is carried out by heating a solution of the amide in an inert organic solvent with a dehydrating agent in the presence of a hydrogen halide acceptor.

The dehydration of the amide in the last step of the reaction scheme can be carried out in an inert organic solvent such as a halogenated hydrocarbon solvent for example, methylene chloride, chloroform, a di- or tri-chloroethane, such as 1,2-dichloroethane and 1,1,2-trichloroethane.

Dehydrating agents which can be used in preparing the 1-cyanomethyl tetrazole include phosphorus oxychloride, phosphorus pentachloride, phosphorus pentoxide or thionyl chloride. Phosphorus oxychloride is the preferred dehydrating agent.

The preparation of the cyanomethyl tetrazole 5-thiol is illustrated in the following reaction scheme.

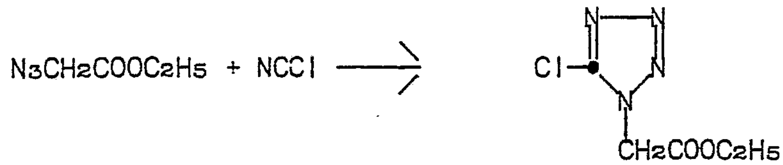

1

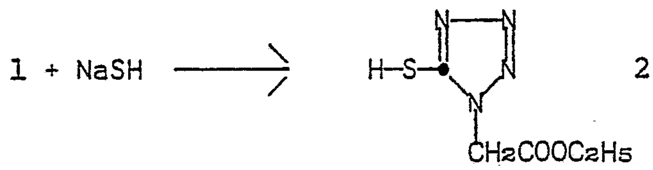

2

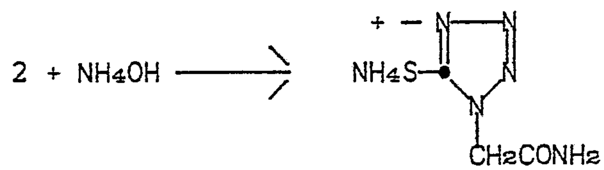

3

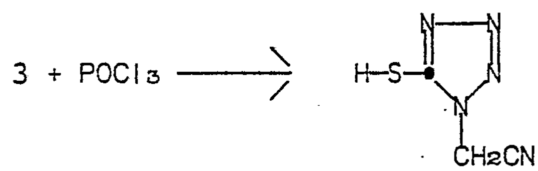

4

The compounds of the invention can be formulated into suitable pharmaceutical formulations for administration. For example, a compound of the formula I or preferable a pharmaceutically acceptable salt thereof can be formulated with a pharmaceutical diluent or carrier such as Water For Injection, Physiological saline and the like for im. injection. Also, the compounds may be administered iv. by the drip method, for example, in a suitable solution such as a glucose solution. Prior to administration the compounds of the invention may be prepared in suitable unit dosage forms in the dry powder state in vials.

The following Examples further describe the present invention. The abbreviations which occur in the Examples have the following meanings: HPLC refers to high performance liquid chromatography; in the designation of the NMR signals, s = singlet, m = multiplet, d = doublet, and q = quartet.

HPLC was carried out on a Waters and Associates Model 500 (silica gel) column.

Nuclear magnetic resonance spectra were obtained on Jeol Model FX-90Q for 90MHz spectra. Trimethylsilane (TMS) was used as the standard in NMR spectra.

Preparation of 1-cyanomethyl-1H-tetrazol-5-ylthiol

A. Ethyl azidoacetate

To a solution of 490 g. (4 moles) of ethyl chloroacetate in 1500 ml. of acetonitrile were added 260 g. (4 moles) of sodium azide, and the mixture was heated at the reflux temperature for 20 hours. After heating, the reaction mixture was poured into 1 liter of water with stirring for 1/2 hour. The organic phase was separated from the aqueous phase and evaporated *in vacuo* to dryness. The yellow residual oil was dissolved in 1200 ml. of diethyl ether and the solution was dried over magnesium sulfate. Evaporation of the diethyl ether *in vacuo* gave 391 g. (76% yield) of ethyl azidoacetate.

B. Ethyl 5-chloro-1H-tetrazol-1-ylacetate

A mixture of 130 g. (1 mole) of ethyl azidoacetate prepared as described in part A and 96 g. (1.56 mole) of cyanogen chloride was heated at a temperature of 125°C. for 20 hours. After the reaction mixture had cooled, the reaction product mixture was dissolved in ethyl acetate, and the solution was filtered and evaporated *in vacuo* yielding a yellow crystalline mass of product. The yellow crystals were recrystallized from aqueous ethyl alcohol and gave 149 g. (78% yield) of ethyl 5-chloro-1H-tetrazol-1-ylacetate as pale yellow crystals melting at about 57-60°C.

C. Ethyl 5-thiol-1H-tetrazol-1-ylacetate

A solution of 209 g. of the chlorotetrazole ester, prepared as described in part B above, and 250 g. of sodium hydrosulfide in 5 liters of ethyl alcohol was heated at the reflux temperature for 24 hours. After heating, the reaction mixture was acidified with concentrated hydrochloric acid, and the volume of the acidified mixture was reduced to 1/4 the original volume by evaporation *in vacuo*. The concentrate was extracted with ethyl acetate, the extract was dried and evaporated to dryness under reduced pressure. The residual product was recrystallized from toluene-methylene chloride-hexane and gave 129 g. of the product melting at about 85°C. to 88°C.

D. 5-Thiol-1H-tetrazol-1-ylacetamide ammonium salt

A solution of 20 g. (0.106 mole) of the tetrazolthiol ester, prepared as described above in part C, in 320 ml. of concentrated ammonium hydroxide and 200 ml. of ethyl alcohol containing 500 ml. of ammonium chloride was heated at the reflux temperature for about 12 hours. After heating, the reaction mixture was evaporated *in vacuo*, and the yellow crystalline residue obtained was recrystallized from hot ethyl alcohol to yield a first crop of 13.7 g. (73% yield) of the product as white crystals melting at about 197 to about 199°C. after vacuum drying. A second crop of 1.4 g. of the product was obtained which melted at about 191-193°C.

E. 1-Cyanomethyl-1H-tetrazol-5-thiol

A suspension of 5.28 g. of the tetrazolamide ammonium salt, prepared as described above in part D, in 90 ml. of methylene chloride containing 14.4 ml. of pyridine was cooled to a temperature of about 0°C. To the suspension was added dropwise with stirring a solution of 4.6 g. (30 mmole) of phosphorous oxychloride in 40 ml. of methylene chloride. After the addition was completed, the reaction mixture was heated at the reflux temperature for 30 minutes and was then cooled to room temperature with stirring. The reaction mixture had turned orange after heating and contained some precipitate. The reaction mixture was evaporated to dryness *in vacuo* and the residue dissolved in ethyl acetate-water, 1:1, v:v. The pH of the solution was

adjusted to pH 2 with 20% aqueous hydrochloric acid. The acidified solution was then extracted twice with 75 ml. portions of ethyl acetate and the extracts combined. The extract was then washed with 5% hydrochloric acid, with brine, was dried over sodium sulfate and evaporated in vacuo. The brown oil obtained as a residue crystallized on standing. The crystals were vacuum dried at room temperature and yielded after drying 2.6 g. (61% yield) of light brown product melting at about 113-114°C.

The above reaction was repeated on a 10.6 g. batch of the tetrazol amide ammonium salt and 3.7 g. of the nitrile as off-white crystals melting at about 116-118°C. were obtained.

The following analytical data were obtained for the crystalline product.

Elemental analysis calcualted for $C_3H_3N_5S$:

Theory: C, 25.53; H, 2.14; N, 49.62.

Found: C, 25.82; H, 2.40; N, 49.91.

The mass spectrum of the crystalline product showed a molecular weight of 141 in agreement with the product.

## EXAMPLE 1

syn 7β-[α-Methoximino-α-(2-amino-1,3-thiazol-4-yl)-acetamido]-3-(1-cyanomethyl-1H-tetrazol-5-ylthiomethyl)-3-cephem-4-carboxylic acid

A solution of 1-cyanomethyl-1H-tetrazol-5-thiol, 280 mg, was obtained in 12 ml of pH 7 buffer by adding 340 mg of sodium bicarbonate to a suspension of the compound in the buffer. After solution was obtained, 800 mg of syn 7β-[α-methoximino-α-(2-amino-1,3-thiazol-4-yl)acetamido]-3-acetoxymethyl-3-cephem-4-carboxylic acid were added to the solution which was then heated in 60°C. for about 3.5 hours. Heating was discontinued and the reaction mixture was stored in the refrigerator for about 12 hours. Analytical HPLC carried out on the reaction mixture showed the presence of about 60% starting material and 40% product. The starting material and product were separated by chromatography of the reaction mixture on reverse phase silica gel at 200 psi using the solvent system 12% acetonitrile:2% acetic acid:86% water, v:v:v. The starting material was collected in fraction 60-70 while the product was collected in fractions 103-120. The fractions containing the starting material were combined and evaporated to dryness to yield 180 mg of starting material. Fractions 103-120 were combined and freeze-dried to yield 107 mg of the title compound as product.

The following physical data were obtained on the above product.

Elemental analysis for $C_{17}H_{16}O_5N_{10}S_3$:
Theory: C, 38.05; H, 3.01; N, 26.10.
Found: C, 37.77; H, 3.00; N, 25.86.

The infrared spectra of the product (KBR) showed an absorption maximum at 1770 $cm^{-1}$ for the β-lactam carbonyl.

Field desorption mass spectrum; $M^+$=537.

NMR (90 MHz, $DMSOd_6$, reference DMSO (2.65 ppm); 3.84 (m, C-2 methylene), 3.96 (s, oxime methyl, syn), 4.50 (m, C-3' methylene), 5.24 ppm (d, C-6 H), 5.90 (q, C-7 H), 4.92 (s, cyanomethyl methylene), 6.87 (s, thiazole H), 7.32 (broad s, thiazole amino H), and 9.68 (d, 7-amide H) ppm, δ.

EXAMPLE 2

7β-(D-Mandelamido)-3-(1-cyanomethyl-1H-tetrazol-5-ylthiomethyl)-3-cephem-4-carboxylic acid

To a solution of 1-cyanomethyl-1H-tetrazole-5-thiol in aqueous tetrahydrofuran containing excess sodium bicarbonate is added 7β-(D-mandelamido)cephalosporanic acid and the solution is heated at a temperature of about 55-65°C. for four hours. The reaction mixture is evaporated to dryness and the residue taken up in fresh water and ethyl acetate layered over the solution. With stirring the mixture is acidified with hydrochloric acid and the aqueous phase separated. The organic phase is washed with water and brine and dried over sodium sulfate. The dried solution is evaporated to dryness to provide the title compound.

EXAMPLE 3

By following the reaction procedures and conditions described in Example 2, 7-(2-thienylacetamido)-cephalosporanic acid (cephalothin), is reacted with 1-cyanomethyl-1H-tetrazole-5-thiol to provide 7-(2-thienylacetamido)-3-(1-cyanomethyl-1H-tetrazol-5-ylthiomethyl)-3-cephem-4-carboxylic acid.

EXAMPLE 4

By following the procedures and under the conditions described in Example 2, 7-phenoxyacetamido-cephalosporanic acid is reacted with 1-cyanomethyl-1H-tetrazole-5-thiol to provide 7-phenoxyacetamido-3-(1-cyanomethyl-1H-tetrazol-5-ylthiomethyl)-3-cephem-4-carboxylic acid.

EXAMPLE 5

7-(4-Chlorophenylmercaptoacetamido)-3-(1-cyanomethyl-1H-tetrazol-5-ylthiomethyl)-3-cephem-4-carboxylic acid is prepared with 7-(4-chlorophenylmercaptoacetamido)cephalosporanic acid and 1-cyano-methyl-1H-tetrazole-5-thiol by following the procedures described by Example 2.

EXAMPLE 6

7-Chloroacetamido-3-(1-cyanomethyl-1H-tetrazol-5-ylthiomethyl)-3-cephem-4-carboxylic acid is prepared by acylating 7-amino-3-(1-cyanomethyl-1H-tetrazol-5-ylthiomethyl)-3-cephem-4-carboxylic acid with chloroacetyl chloride in aqueous acetone in the presence of sodium carbonate.

## CLAIMS

1. A compound of the formula

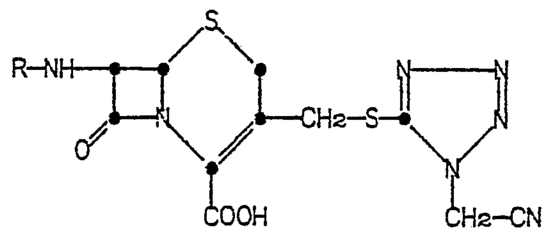

I

wherein R is hydrogen or an acyl group of the formula

$$R'-\overset{O}{\overset{\|}{C}}-$$

wherein R' is hydrogen, $C_1$-$C_4$ alkyl, or $C_1$-$C_4$ alkyl substituted by halogen or cyano; or R' is a group of the formula

$$R_1-\underset{b}{\overset{a}{C}}-$$

wherein $R_1$ is phenoxy, phenylthio, phenyl, hydroxyphenyl, halophenyl, halo-substituted-hydroxyphenyl, or $C_1$-$C_4$ alkylphenyl, thienyl, furyl, or 2-aminothiazol-4-yl, a is hydrogen; b is hydrogen, hydroxy or amino, and a and b when taken together form an oximino group of the formula

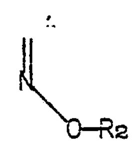

wherein $R_2$ is hydrogen, $C_1$-$C_4$ alkyl or a group of the formula

$$-\overset{\displaystyle R_3}{\underset{\displaystyle R_4}{\overset{|}{\underset{|}{C}}}}-COOR_5$$

wherein $R_3$ and $R_4$ independently are hydrogen, $C_1$-$C_4$ alkyl, or $R_3$ and $R_4$ when taken together with the carbon atom to which they are attached form a four, five or six-membered carbocyclic ring; $R_5$ is hydrogen or a carboxy protecting group;

and the pharmaceutically acceptable salts thereof.

2. The compound of claim 1 wherein R is hydrogen.

3. The compound of claim 1 wherein R is an acyl group $R'-\overset{O}{\overset{\|}{C}}-$ .

4. The compound of claim 3 wherein R' is a group of the formula

$$R_1-\overset{\displaystyle a}{\underset{\displaystyle b}{\overset{|}{\underset{|}{C}}}}- \quad .$$

5. The compound of claim 4 wherein a and b are both hydrogen.

6. The compound of claim 4 wherein a is hydrogen and b is hydroxy or amino.

7. The compound of claim 4 wherein a and b together form an oximino group.

8. The compound of claim 5, 6 or 7 wherein $R_1$ is phenyl or 2-furyl.

9. The compound of claim 7 wherein $R_1$ is 2-amino-1,3-thiazol-4-yl.

10. The compound of claim 9 of the formula

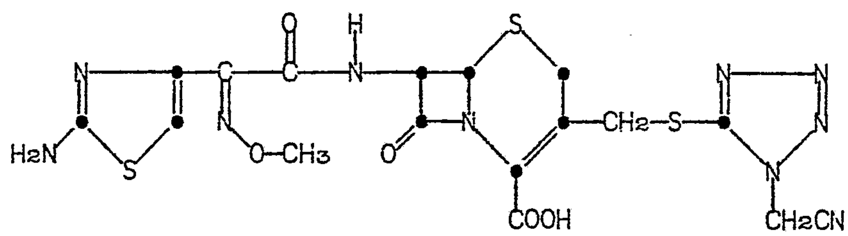

and the pharmaceutically acceptable salts thereof.

11. An antibiotic formulation comprising a compound of the formula I wherein R is an acyl group $R'-\overset{O}{\overset{\parallel}{C}}-$, and a pharmaceutical carrier.

12. The formulation of claim 11 wherein R' is a group of the formula

$$R_1-\overset{a}{\underset{b}{C}}- .$$

13. The formulation of claim 12 wherein a and b together form an oximino group.

14. The formulation of claim 12 comprising the antibiotic of the formula

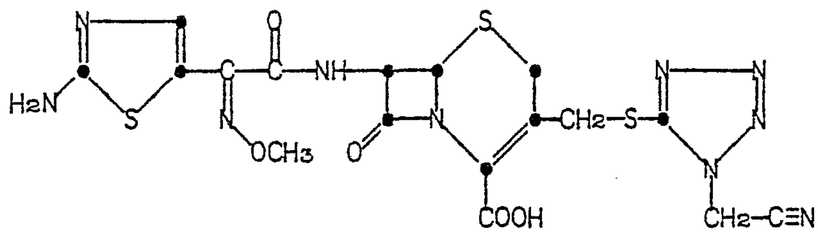

or a pharmaceutically acceptable salt thereof.

15. A process for the preparation of a compound of the formula

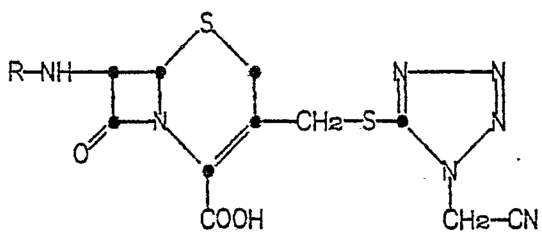

I

wherein R is hydrogen or an acyl group of the formula

$$R'-\overset{\overset{\displaystyle O}{\|}}{C}-$$

wherein R' is hydrogen, $C_1$-$C_4$ alkyl, or $C_1$-$C_4$ alkyl substituted by halogen or cyano; or R' is a group of the formula

$$R_1-\underset{\underset{\displaystyle b}{|}}{\overset{\overset{\displaystyle a}{|}}{C}}-$$

wherein $R_1$ is phenoxy, phenylthio, phenyl, hydroxyphenyl, halophenyl, halo-substituted-hydroxyphenyl, or $C_1$-$C_4$ alkylphenyl, thienyl, furyl, or 2-aminothiazol-4-yl,

a is hydrogen;

b is hydrogen, hydroxy or amino, and a and b when taken together form an oximino group of the formula

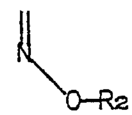

wherein $R_2$ is hydrogen, $C_1$-$C_4$ alkyl or a group of the formula

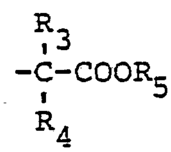

wherein $R_3$ and $R_4$ independently are hydrogen, $C_1$-$C_4$ alkyl, or $R_3$ and $R_4$ when taken together with the carbon atom to which they are attached form a four, five or six-membered carbocyclic ring; $R_5$ is hydrogen or a carboxy protecting group;

and the pharmaceutically acceptable salts thereof which comprises reacting a compound of the formula

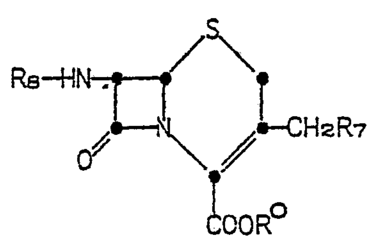

II

wherein $R_7$ is $C_1$-$C_4$ acyloxy or halo, $R^0$ is hydrogen or a carboxylic acid protecting ester group,

and $R_8$ is hydrogen or $R'-\overset{O}{\overset{\|}{C}}-$, wherein R' is defined above;

with a 1-cyanomethyl-1H-tetrazole-5-thiol compound of the formula

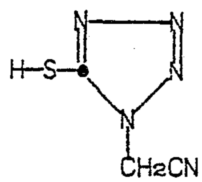

III

in an inert organic solvent.

16. The process of claim 15 wherein the starting material is a compound of formula II wherein $R_7$ is acetoxy, $R_8$ is $R'-\overset{O}{\overset{\|}{C}}-$ wherein R' is the same as defined for formula I and $R^0$ is hydrogen.

17. A process of claim 15 wherein the starting material is a compound of formula II wherein $R_7$ is acetoxy and $R_8$ and $R^0$ are both hydrogen.

18. A process of claim 17 wherein the resulting 1-cyanomethyl-1H-tetrazole-5-thiol substituted nucleus is acylated with a carboxylic acid of the formula R'COOH, wherein R' is the same as defined in formula I.

19. A process of claim 18 wherein the resulting 1-cyanomethyl-1H-tetrazole-5-thiol substituted nucleus is acylated with an active derivative of the carboxylic acid of the formula R'COX wherein R' is the same as defined for formula I and X is chloro, bromo, azido, or an active ester forming moiety.

20. A process of claim 15 wherein the starting material is a compound of formula II wherein $R_7$ is chloro, bromo or iodo, $R_8$ is $R'-\overset{O}{\overset{\parallel}{C}}-$ wherein R' is the same as defined in formula I and $R^0$ is a carboxylic acid protecting ester group.

21. The process of claim 20 wherein the reaction is carried out in the presence of a hydrogen halide acceptor.

22. The process of claim 20 wherein the carboxylic acid protecting ester group is removed to provide the corresponding acid.

23. A compound according to any of claims 1 to 10, for use as an antibiotic.

CLAIMS

1. A process for the preparation of a compound of the formula

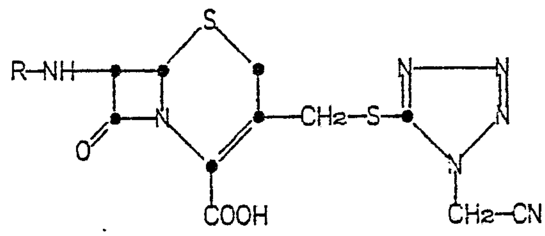

I

wherein R is hydrogen or an acyl group of the formula

$$R'-\overset{O}{\overset{\|}{C}}-$$

wherein R' is hydrogen, $C_1$-$C_4$ alkyl, or $C_1$-$C_4$ alkyl substituted by halogen or cyano; or R' is a group of the formula

$$R_1-\underset{b}{\overset{a}{C}}-$$

wherein $R_1$ is phenoxy, phenylthio, phenyl, hydroxyphenyl, halophenyl, halo-substituted-hydroxyphenyl, or $C_1$-$C_4$ alkylphenyl, thienyl, furyl, or 2-aminothiazol-4-yl,

a is hydrogen;

b is hydrogen, hydroxy or amino, and a and b when taken together form an oximino group of the formula

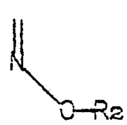

wherein $R_2$ is hydrogen, $C_1$-$C_4$ alkyl or a group of the formula

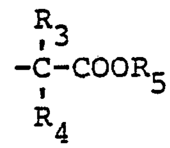

wherein $R_3$ and $R_4$ independently are hydrogen, $C_1$-$C_4$ alkyl, or $R_3$ and $R_4$ when taken together with the carbon atom to which they are attached form a four, five or six-membered carbocyclic ring; $R_5$ is hydrogen or a carboxy protecting group;

and the pharmaceutically acceptable salts thereof characterized in that a compound of the formula

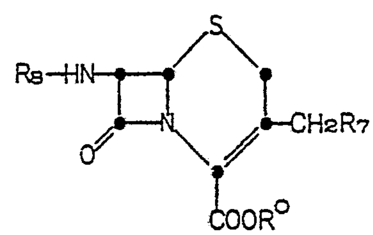

II

wherein $R_7$ is $C_1$-$C_4$ acyloxy or halo, $R^0$ is hydrogen or a carboxylic acid protecting ester group, and $R_8$ is hydrogen or $R'-\overset{O}{\overset{\|}{C}}-$, wherein R' is defined above;

is reacted with a 1-cyanomethyl-1H-tetrazole-5-thiol compound of the formula

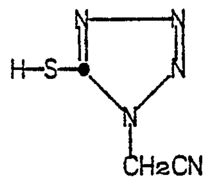

III

in an inert organic solvent.

2. The process of claim 1 characterized in that the starting material is a compound of formula II wherein $R_7$ is acetoxy, $R_8$ is $R'-\overset{O}{\overset{\|}{C}}-$ wherein R' is the same as defined for formula I and $R^0$ is hydrogen.

3. The process of claim 2 characterized in that the reaction temperature is 35°C. to about 75°C.

4. The process of claim 2 or 3 characterized in that the pH of the reaction is about 7 to about 9.

5. A process of claim 1 characterized in that the starting material is a compound of formula II wherein $R_7$ is acetoxy and $R_8$ and $R^0$ are both hydrogen.

6. A process of claim 5 characterized in that the reaction temperature is about 40°C. to about 75°C.

7. A process of claim 5 or 6 characterized in that the reaction temperature is about 60°C.

8. A process of claim 5, 6 or 7 characterized in that the pH of the reaction is about 7 to about 9.

9. A process of anyone of claims 5 to 8 characterized in that the resulting 1-cyanomethyl-1H-tetrazole-5-thiol substituted nucleus is acylated with a carboxylic acid of the formula R'COOH, wherein R' is the same as defined in formula I.

10. A process of claim 9 characterized in that the resulting 1-cyanomethyl-1H-tetrazole-5-thiol substituted nucleus is acylated with an active derivative of the carboxylic acid of the formula R'COX wherein R' is the same as defined for formula I and X is chloro, bromo, azido, or an active ester forming moiety.

11. A process of claim 1 characterized in that the starting material is a compound of formula II wherein $R_7$ is chloro, bromo or iodo, $R_8$ is $R'-\overset{O}{\overset{\|}{C}}-$ wherein R' is the same as defined in formula I and $R^0$ is a carboxylic acid protecting ester group.

12. The process of claim 11 characterized in that the reaction is carried out in the presence of a hydrogen halide acceptor.

13. The process of claim 11 characterized in that the carboxylic acid protecting ester group is removed to provide the corresponding acid.